# FASCICULE DE BREVET EUROPEEN

(11) **EP 0 737 063 B1**
(45) Date de publication et mention de la délivrance du brevet: **14.04.1999**
(21) Numéro de dépôt: 95906369.4
(22) Date de dépôt: 05.01.1995
(51) Int. Cl.: A61K 9/127, A61K 7/00

(54) **PROCEDE DE PREPARATION DE LIPOSOMES SANS UTILISATION DE SOLVANT ORGANIQUE**
VERFAHREN TUR LIPOSOMENHERSTELLUNG OHNE VERWENDUNG ORGANISCHER LOESUNGSMITTEL
METHOD FOR PREPARING LIPOSOMES WITHOUT USING AN ORGANIC SOLVENT

(30) Priorité: 06.01.1994 FR 9400090
(43) Date de publication de la demande: 16.10.1996
(73) Titulaire: CAPSULIS, 33600 Pessac (FR)
(72) Inventeur: ROUX, Didier, F-33700 Mérignac (FR); DEGERT, Corinne, F-33160 Saint-Médard-en-Jalles (FR); LAVERSANNE, René, F-33600 Pessac (FR)
(74) Mandataire: Giraud, Françoise
(86) Numéro de dépôt international: FR9500012
(87) Numéro de publication internationale: WO9518601

(56) Documents cités:
- EP-A- 0 220 797
- EP-A- 0 299 937
- WO-A-88/06882
- WO-A-90/00399
- WO-A-91/04013
- WO-A-93/19735
- DATABASE WPI Week 9312 Derwent Publications Ltd., London, GB; AN 93-096927 & JP,A,05 039 484 (KOSE KK) , 19 Février 1993

## Description

La présente invention concerne un procédé de préparation de liposomes sans utilisation de solvant organique.

Les liposomes comprennent habituellement un tensioactif lipidique ainsi que du cholestérol et/ou un/des produit(s) actif(s) encapsulé(s) . Le cholestérol (plus généralement un stérol) se lie avec le phospholipide pour améliorer les propriétés élastiques et d'imperméabilité de la membrane lipidique. Si le cholestérol ou le produit actif à encapsuler sont mélangés directement avec le lipide et le solvant aqueux, le mélange reste inhomogène. Par exemple, on peut observer que le cholestérol reste après plusieurs jours sous forme de petits cristaux qui ne se dissolvent pas dans la préparation même par adjonction d'un grand excès d'eau. Afin de pallier à ce problème on passe par un procédé dit "d'atomisation" (voir EP-A-87 993) qui comprend le mélange des différents constituants dans un solvant organique (généralement chloré tel que le chloroforme ou le dichlorométhane, le méthanol est aussi utilisé) avant leur utilisation en milieu aqueux. L'évaporation de ce solvant produit une poudre qui est ensuite utilisée directement dans le procédé de préparation des liposomes (voir par exemple EP-A-0 107 559). L'inconvénient de ce procédé est que le passage par l'atomisation peut laisser des traces (petites mais impossible à faire disparaître) de solvant dans la préparation finale. Compte tenu des effets nuisibles sur la santé de telles traces il est indispensable d'arriver à préparer des liposomes sans utiliser de solvant organique.

Des procédés semblables sont utilisés pour incorporer dans des liposomes des protéines membranaires, c'est-à-dire des protéines solubles dans les membranes mais difficilement solubles dans l'eau.

La présente invention vise à fournir un procédé de préparation de liposomes permettant de se dispenser de l'étape d'atomisation. L'originalité du procédé repose sur l'utilisation d'un co-tensioactif bien choisi qui permet de solubiliser le cholestérol ou le produit actif à encapsuler sans utiliser un solvant organique.

La présente invention a ainsi pour objet un procédé de préparation de liposomes comprenant :
1) le mélange d'un solvant aqueux, d'un tensioactif comprenant une tête hydrophile et une chaîne hydrocarbonée en C₂ à C₁₆, d'un stérol et/ou une protéine membranaire et/ou d'un produit à encapsuler de façon à obtenir une dissolution du stérol et/ou du produit à encapsuler;
2) le mélange de la composition ainsi obtenue avec un tensioactif lipidique de façon à former une phase cristal liquide lamellaire homogène ou une suspension de phase cristal liquide dans de l'eau, et
3) la transformation de la phase cristal liquide ou de la suspension de phase cristal liquide en liposomes.

Il est à noter que dans certains cas la transformation de la phase cristal liquide en liposomes se produit spontanément.

La taille des liposomes spontanés est typiquement de 100 à 500 nm plus généralement de 50 à 5000 nm. Les liposomes spontanés obtenus sont des liposomes multilamellaires comportant généralement de 10 à 10000 couches alternées d'eau et de tensioactifs (à titre hydrophile et lipophiles) y compris au coeur des liposomes.

Mais l'invention a également pour objet la phase cristal liquide ou la suspension de phase cristal liquide obtenue au deuxième stade.

On décrira maintenant de façon plus détaillée la présente invention.

Le tensioactif utilisé au premier stade comprend une chaîne carbonée en C₂ à C₁₆ ramifiée ou non et de préférence en C₆ à C₁₄.

La tête hydrophile est généralement un alcool ou un alcool éthoxylé mais peut être aussi un acide carboxylique ou un sel d'acide gras, un ammonium quaternaire, un sulfonate ou un sulfate ou tout autre groupement ionique ou non ionique polaire.

Dans le premier stade, le solvant aqueux, le tensioactif et le cholestérol et/ou une protéine membranaire et/ou le produit à encapsuler sont mélangés en proportions adéquates. Généralement, le tensioactif est utilisé en une proportion de 1 à 50 % en poids, de préférence de 5 à 25 % et le cholestérol en une proportion de 0 % à 25 % en poids. Après agitation, généralement le mélange est laissé 1 à 2 heures au repos. On peut dans certains cas solubiliser le cholestérol dans ce mélange avec ou sans chauffage (40 à 100° Celsius) et agitation. Cette solubilisation peut durer de quelques heures à quelques jours. Il est possible de solubiliser aussi dans certains cas d'abord le cholestérol dans le tensioactif lorsqu'il est liquide (alcool, alcool éthoxylé, etc...) puis d'ajouter le solvant aqueux.

Dans le deuxième stade, le tensioactif lipidique est ajouté à la composition obtenue au premier stade en proportion nécessaire pour la formation d'une phase cristal liquide ou d'une suspension de phase cristal liquide dans l'eau. Typiquement on ajoute de 1 à 50 %, de préférence de 5 à 25 % en poids de tensioactif lipidique. De préférence, le tensioactif lipidique est ajouté en une quantité supérieure à celle du tensioactif utilisé au premier stade. Le tensioactif lipidique est choisi parmi ceux classiquement utilisés pour la fabrication des liposomes et est généralement un phospholipide. Généralement le mélange est agité doucement pendant 5 à 48 heures. Si nécessaire on peut le porter à une température plus élevée de l'ordre de 60-80° Celsius afin d'accélérer le processus de dissolution. Puis le mélange est ramené à température ambiante. Dans certains cas, on peut ajouter le produit à encapsuler à ce moment, juste avant d'ajouter le tensioactif lipidique.

On obtient ainsi une phase cristal liquide lamellaire homogène ou une suspension de phase cristal liquide dans un excès d'eau. L'ensemble du cholestérol et/ou du produit actif à encapsuler ont été solubilisés. On peut alors utiliser cette pâte pour préparer des liposomes selon une méthode classique (ultrasonication, extrusion) ou par cisaillement constant selon le procédé décrit dans FR-A 2 689 418.

Comme indiqué précédemment, dans certains cas, on obtient la formation de sphérulites spontanées sans le besoin d'étapes supplémentaires. Ces sphérulites peuvent être facilement caractérisées par microscopie optique à contraste de phase ou ordinaire lorsque leur taille est suffisamment importante et/ou par cryofracture et microscopie électronique. Lorsque le dosage conduit à la formation de sphérulites spontanées, la crème obtenue après le second stade peut être diluée directement dans une solution aqueuse et les liposomes initialement empilés de façon compacte se séparent et restent en suspension diluée. Lorsqu'un produit actif a été incorporé lors de la première étape, celui-ci se retrouve à l'intérieur des liposomes.

Il est à noter que par ailleurs on peut disperser les liposomes obtenus dans une phase huileuse. Dans ce cas, la dispersion est homogène après une agitation de quelques minutes. Cependant, au bout de quelques heures, les liposomes finissent par décanter mais peuvent être remis en suspension par simple agitation. La phase huileuse que l'on peut utiliser peut être entre autres une huile minérale ou une huile végétale.

Le procédé selon l'invention permet d'incorporer une substance active du point de vue pharmaceutique ou cosmétique, substance qui peut être incorporée suivant sa nature, soit dans les parties aqueuses, soit dans les parties formées par les tensioactifs.

A titre d'exemples de substances actives, on peut citer :
- dihydroxyacétone (DHA),
- alpha hydroxy acides (acide de fruits) et plus spécifiquement acides glycoliques, lactiques, tartriques, salyciliques,
- filtres solaires hydrosolubles et liposolubles,
- huiles essentielles,
- composés insaponifiables,
- hyaluronate de sodium,
- TiO₂ micronisé,
- céramides,
- caféine,
- vitamines A, E et C.

Les exemples suivants illustrent la présente invention.

### EXEMPLE 1

1,5 g de pentaoxyethylene lauryl éther (C12E5) de la société NIKKOL est dissous dans 7g d'eau. On ajoute 0,14 g de cholestérol (MERCK). On mélange et l'on peut clairement voir avec les yeux et sous microscope optique les cristaux de cholestérol non dissous. On porte le mélange à 80° pendant 12 heures afin d'obtenir une complète dissolution du cholestérol dans le mélange eau/C12E5. On peut éventuellement agiter de temps en temps le mélange ou de façon permanente, le temps de dissolution du cholestérol est alors réduit. Le mélange homogène obtenu est ramené à température ambiante. On ajoute alors 1,35 g de lécithine de soja (SIGMA) puis on mélange et après 12 h d'attente on obtient une phase cristal liquide lamellaire homogène.

On peut vérifier soit par microscopie optique après dilution soit par cryofracture et microscopie électronique sur la crème pure que le mélange est constitué de liposomes concentrés de rayon d'environ 200 à 500 nm. On peut afin d'améliorer l'homogénéité en taille, traiter directement le mélange obtenu (avant dilution) selon le procédé décrit dans FR-A-2 689 418.

### EXEMPLE 2

On opère comme à l'exemple précédent en suivant le même ordre mais avec les proportions suivantes:
2,5 g de C12E5
2,1 g de lécithine
0,4 g de cholestérol
5 g d'eau.

On obtient spontanément des liposomes à l'issue du deuxième stade.

### EXEMPLE 3

On opère comme à l'exemple 1 mais avec les proportions suivantes :
0,5 g de C12E5
2,5 g de lécithine de soja
0,25 g de cholestérol
6,75 g d'eau.

Il est cependant nécessaire d'attendre au moins 24 h à 60° pour obtenir un mélange homogène d'eau, de C12E5 et de cholestérol.

On obtient spontanément des liposomes à l'issue du deuxième stade.

### EXEMPLE 4

On opère comme à l'exemple 1, mais on remplace le C12E5 de la société NIKKOL par du LAUROPAL de la société WITCO (constitué principalement de C12E5) et en utilisant les proportions suivantes :
1,1 g de LAUROPAL 0205
1,9 g de lécithine de soja
0,2 g de cholestérol
6,8 g d'eau
ou
0,4 g de LAUROPAL 0205
2,6 g de lécithine de soja
0,25 g de cholestérol
6,8 g d'eau.

### EXEMPLE 5

On opère comme à l'exemple 1 mais en utilisant un autre tensioactif non-ionique : LAUROPAL 4 de la société WITCO (constitué principalement de C12E4) et les proportions suivantes :
1,5 g de LAUROPAL 4
2,5 g de lécithine de soja
0,25 g de cholestérol
6 g d'eau
ou
0,7 g de LAUROPAL 4
3,3 g de lécithine de soja
0,33 g de cholestérol
6 g d'eau.

### EXEMPLE 6

On opère comme à l'exemple 1 en utilisant un autre tensioactif non-ionique : REMCOPAL 121 de la société CECA (constitué principalement de C12E3) et les proportions suivantes :
0,7 g de REMCOPAL 121
3 g de lécithine de soja
0,33 g de cholestérol
6 g d'eau
ou
0,5 g de REMCOPAL 121
3 g de lécithine de soja
0,35 g de cholestérol
6 g d'eau.

### EXEMPLE 7

On opère comme à l'exemple 1 en utilisant :
1 g d'oléate de potassium (Aldrich)
3 g de lécithine de soja
0,3 g de cholestérol
6 g d'eau
ou
0,5 g d'oléate de potassium
3,5 g de lécithine de soja
0,35 g de cholestérol
6 g d'eau.

### EXEMPLE 8

On incorpore dans l'eau de préparation de l'exemple 4 (deuxième composition) une concentration de 10⁻³ molaire de calcéine. On peut après préparation vérifier que la sonde fluorescente (calcéine) a bien été incorporée en mesurant la cinétique de déclin de fluorescence par extinction avec du cobalt.

### EXEMPLE 9

On met dans un erlenmeyer 2,5 g de Remcopal 121 (CECA) et 0,8 g de cholestérol (Aldrich). On ajoute 4 g d'eau, mélange puis laisse reposer pendant 2 à 3 heures à 65° C jusqu'à dissolution complète du cholestérol, on laisse refroidir à température ambiante. On ajoute 10 g de lécithine Phospholipon 90P (Nattermann), on mélange pendant quelques minutes, ajoute goutte à goutte 8,5 g d'une solution aqueuse contenant 40 % en poids de DHA (Merck) et ajuste à pH3 avec de l'acide chlorhydrique. On mélange intimement afin d'obtenir une pâte homogène et laisse reposer 24 h à température ambiante.

On peut ensuite diluer cette pâte dans de l'eau pour obtenir une suspension homogène de capsules contenant la DHA. On ajuste ensuite le pH à pH = 5, ce qui permet d'avoir une formulation externe des capsules à ce pH tout en gardant la DHA dans les capsules à un pH plus acide. Des mesures de pH et des dosages en fonction du temps permettent de vérifier que la dégradation de la DHA est ralentie lorsque celle-ci est microencapsulée par rapport à une solution aqueuse simple.

### EXEMPLE 10

On met dans un erlenmeyer 2,5 g de Remcopal 121 et 0,8 g de cholestérol. On ajoute 4 g d'eau, mélange puis laisse reposer pendant 2 à 3 heures à 65° C jusqu'à dissolution complète du cholestérol. On laisse refroidir à température ambiante. On ajoute 10 g de lécithine Phospholipon 90P et 1 g d'acide salicylique (Aldrich). On mélange pendant quelques minutes, ajoute goutte à goutte 7 g d'une solution aqueuse contenant 42 mg de parahydroxybenzoate de méthyle sodés, 89 mg de paraphydroxybenzoate de propyle sodés et 127 mg de 2-phénoxy éthanol (Aldrich). On mélange pendant quelques minutes et laisse reposer 24 h à 37° C. Cette pâte homogène peut ensuite être dispersée dans de l'eau pour former des liposomes multilamellaires contenant l'acide salicylique. Une séparation par centrifugation puis un dosage permet de montrer que plus de 70 % de l'acide est contenu dans les capsules. L'ajout d'un indicateur coloré à la préparation permet de montrer en effectuant un dosage acido-basique que l'intérieur des capsules est à un pH de 2 unités de pH environ plus faible que l'extérieur.

### EXEMPLE 11

On met dans un erlenmeyer 2,5 g de Remcopal 121 et 0,8 g de cholestérol. On ajoute 4 g d'eau, mélange puis laisse reposer pendant 2 à 3 heures à 65° C jusqu'à dissolution complète du cholestérol, laisse refroidir à température ambiante. On ajoute 10 g de lécithine Phospholipon 90P. On mélange pendant quelques minutes, ajoute goutte à goutte 8,5 g d'une solution aqueuse contenant 1 % de hyaluronate de sodium (Laboratoire Bomann). On laisse reposer 24 h. On peut disperser ce mélange dans de l'eau afin d'obtenir une suspension aqueuse de capsules contenant le hyaluronate de sodium.

On peut également disperser le mélange dans de l'huile de ricin et obtenir une suspension homogène de capsules qui peut être utilisée dans des préparations futures. Cependant, au bout de quelques heures les capsules décantent mais peuvent être remises en suspension par simple agitation.

### EXEMPLE 12 (filtre solaire soluble dans l'eau).

On prépare une phase aqueuse contenant 10 % d'Eusolex 232 (Merck) : phase A.

On met dans un erlenmeyer 2,3 g de Remcopal 121 et 0,8 g de cholestérol, ajoute 4 g d'eau, on mélange puis laisse reposer pendant 2 à 3 heures à 65° C jusqu'à dissolution complète du cholestérol, on laisse refroidir à température ambiante. On ajoute 11,3 g de lécithine Phospholipon 25P (Nattermann). On mélange pendant quelques minutes, ajoute goutte à goutte 7 g de phase A. On laisse reposer 24 h. La préparation peut être dispersée dans de l'eau ou de l'huile afin d'obtenir une suspension de capsules contenant le filtre solaire.

### EXEMPLE 13 (filtre solaire soluble dans l'huile).

On met dans un erlenmeyer 6 g de Remcopal 121 et 1,5 g de cholestérol, ajoute 8 g d'eau, on mélange puis laisse reposer pendant 2 à 3 heures à 65° C jusqu'à dissolution complète du cholestérol. On laisse refroidir à température ambiante. On ajoute 18 g de lécithine Phospholipon 25P. On ajoute 6 g de méthoxycinnamate, mélange pendant quelques minutes, ajoute goutte à goutte 16 g d'eau. On laisse reposer 24 h, la préparation peut être dispersée dans de l'eau afin d'obtenir une suspension de capsules contenant le filtre solaire.

## Revendications

1. Procédé de préparation de liposomes comprenant :
1) le mélange d'un solvant aqueux, d'un tensioactif comprenant une tête hydrophile et une chaîne hydrocarbonée en C₂ à C₁₆, d'un stérol et/ou une protéine membranaire et/ou d'un produit à encapsuler de façon à obtenir une dissolution du stérol et/ou du produit à encapsuler;
2) le mélange de la composition ainsi obtenue avec un tensioactif lipidique de façon à former une phase cristal liquide lamellaire homogène ou une suspension de phase cristal liquide dans de l'eau, et
3) la transformation de la phase cristal liquide ou de la suspension de phase cristal liquide en liposomes.

2. Procédé de préparation de liposomes selon la revendication 1, dans lequel le tensioactif utilisé au premier stade a une chaîne hydrocarbonée en C₆ à C₁₄.

3. Procédé de préparation de liposomes selon la revendication 1 ou 2, dans lequel au premier stade on utilise un mélange comprenant 1 à 50 % en poids de tensioactif et 0 à 25 % en poids de cholestérol.

4. Procédé de préparation de liposomes selon la revendication 3, dans lequel au premier stade on utilise un mélange comprenant 5 à 25 % en poids de tensioactif.

5. Procédé selon l'une quelconque des revendications précédentes, dans lequel au deuxième stade on ajoute 1 à 50 % en poids de tensioactif lipidique.

6. Procédé selon la revendication 5, dans lequel au deuxième stade on ajoute 5 à 25 % en poids de tensioactif lipidique.

7. Procédé selon l'une quelconque des revendications précédentes, dans lequel le tensioactif lipidique est ajouté en une quantité supérieure à celle du tensioactif utilisé au premier stade.

8. Procédé selon l'une quelconque des revendications précédentes, dans lequel les liposomes obtenus sont dispersés dans une phase huileuse.

9. Procédé de préparation d'une phase cristal liquide ou d'une suspension de phase cristal liquide qui comprend :
1) le mélange d'un solvant aqueux, d'un tensioactif comprenant une tête hydrophile et une chaîne hydrocarbonée en C₂ à C₁₆, d'un stérol et/ou une protéine membranaire et/ou d'un produit à encapsuler de façon à obtenir une dissolution du stérol et/ou du produit à encapsuler;
2) le mélange de la composition ainsi obtenue avec un tensioactif lipidique de façon à former une phase cristal liquide lamellaire homogène ou une suspension de phase cristal liquide dans de l'eau.

## Claims

1. Process for the preparation of liposomes comprising:
1) the mixing of an aqueous solvent, of a surfactant comprising a hydrophilic head and a (C₂-C₁₆) hydrocarbon chain, of a sterol and/or a membrane protein and/or of a product to be encapsulated, so as to obtain dissolution of the sterol and/or of the product to be encapsulated;
2) the mixing of the composition thus obtained with a lipid surfactant, so as to form a homogeneous lamellar liquid crystal phase or a liquid crystal phase suspension in water, and
3) the conversion of the liquid crystal phase or of the liquid crystal phase suspension into liposomes.

2. Process for the preparation of liposomes according to Claim 1, in which the surfactant used in the first stage has a (C₆-C₁₄) hydrocarbon chain.

3. Process for the preparation of liposomes according to Claim 1 or 2, in which, in the first stage, a mixture comprising 1 to 50% by weight of surfactant and 0 to 25% by weight of cholesterol is used.

4. Process for the preparation of liposomes according to Claim 3, in which, in the first stage, a mixture comprising 5 to 25% by weight of surfactant is used.

5. Process according to any one of the preceding claims, in which, in the second stage, 1 to 50% by weight of lipid surfactant is added.

6. Process according to Claim 5, in which, in the second stage, 5 to 25% by weight of lipid surfactant is added.

7. Process according to any one of the preceding claims, in which the lipid surfactant is added in an amount greater than that of the surfactant used in the first stage.

8. Process according to any one of the preceding claims, in which the liposomes obtained are dispersed in an oily phase.

9. Process for the preparation of a liquid crystal phase or of a liquid crystal phase suspension which comprises:
1) the mixing of an aqueous solvent, of a surfactant comprising a hydrophilic head and a (C₂-C₁₆) hydrocarbon chain, of a sterol and/or a membrane protein and/or of a product to be encapsulated, so as to obtain dissolution of the sterol and/or of the product to be encapsulated;
2) the mixing of the composition thus obtained with a lipid surfactant, so as to form a homogeneous lamellar liquid crystal phase or a liquid crystal phase suspension in water.

## Patentansprüche

1. Verfahren zur Herstellung von Liposomen, das umfaßt:
1) das Mischen eines wäßrigen Lösungsmittels, eines Tensids, das einen hydrophilen Kopf und eine C₂-C₁₆-Kohlenwasserstoffkette umfaßt, eines Sterins und/oder eines Membran-Proteins und/oder eines Produkts, das eingekapselt werden soll, zur Erzielung einer Auflösung des Sterins und/oder des einzukapselnden Produkts;
2) das Mischen der so erhaltenen Zusammensetzung mit einem Lipid-Tensid zur Bildung einer homogenen lamellaren Flüssigkristallphase oder einer Suspension der Flüssigkristallphase in Wasser und
3) das Umwandeln der Flüssigkristallphase oder der Flüssigkristallphasen-Suspension in Liposome.

2. Verfahren zur Herstellung von Liposomen nach Anspruch 1, bei dem das in der ersten Stufe verwendete Tensid eine C₆-C₁₄-Kohlenwasserstoffkette aufweist.

3. Verfahren zur Herstellung von Liposomen nach Anspruch 1 oder 2, bei dem man in der ersten Stufe eine Mischung verwendet, die 1 bis 50 Gew.-% Tensid und 0 bis 25 Gew.-% Cholesterin umfaßt.

4. Verfahren zur Herstellung von Liposomen nach Anspruch 3, bei dem man in der ersten Stufe eine Mischung verwendet, die 5 bis 25 Gew.-% Tensid umfaßt.

5. Verfahren nach einem der vorhergehenden Ansprüche, bei dem man in der zweiten Stufe 1 bis 50 Gew.-% Lipid-Tensid zugibt.

6. Verfahren nach Anspruch 5, bei dem man in der zweiten Stufe 5 bis 25 Gew.-% Lipid-Tensid zugibt.

7. Verfahren nach einem der vorhergehenden Ansprüche, bei dem man das Lipid-Tensid in einer Menge zugibt, die höher ist als diejenige des in der ersten Stufe verwendeten Tensids.

8. Verfahren nach einem der vorhergehenden Ansprüche, bei dem die erhaltenen Liposome in einer öligen Phase dispergiert werden.

9. Verfahren zur Herstellung einer Flüssigkristallphase oder einer Flüssigkristallphasen-Suspension, das umfaßt
1) das Mischen eines wäßrigen Lösungsmittels, eines Tensids, das einen hydrophilen Kopf und eine C₂-C₁₆-Kohlenwasserstoffkette umfaßt, eines Sterins und/oder eines Membran-Proteins und/oder eines Produkts, das eingekapselt werden soll, zur Erzielung einer Auflösung des Sterins und/oder des einzukapselnden Produkts; und
2) das Mischen der so erhaltenen Zusammensetzung mit einem Lipid-Tensid zur Bildung einer homogenen lamellaren Flüssigkristallphase oder einer Suspension der Flüssigkristallphase in Wasser.
